Europäisches Patentamt

European Patent Office

Office européen des brevets

(11) Publication number: **0 309 898 A2**

(12) **EUROPEAN PATENT APPLICATION**

(21) Application number: 88115535.2

(51) Int. Cl.⁴: **A61K 39/395 , A61K 39/00**

(22) Date of filing: 22.09.88

(30) Priority: 28.09.87 GB 8722741

(43) Date of publication of application:
05.04.89 Bulletin 89/14

(84) Designated Contracting States:
AT BE CH DE FR GB IT LI LU NL SE

(71) Applicant: **F. HOFFMANN-LA ROCHE & CO. Aktiengesellschaft**

**CH-4002 Basel(CH)**

(72) Inventor: **Stärz, Uwe Dieter, Dr.**
**89 Grienstrasse**
**CH-4055 Basle(CH)**

(74) Representative: **Mezger, Wolfgang, Dr. et al**
**Grenzacherstrasse 124 Postfach 3255**
**CH-4002 Basel(CH)**

(54) Method for the induction of a cytotoxic T cell response.

(57) The present invention relates to a method for the induction of a cytotoxic T cell response characterized in that said response is induced by a soluble antigen derived from a pathogenic agent, to a vaccine to be used for the induction of a cytotoxic T cell response characterized in that it contains a soluble antigen derived from a pathogenic agent and to a method for the preparation of said vaccine. The invention further relates to the use of a soluble antigen derived from a pathogenic agent for inducing a cytotoxic T cell response.

# Fig. 1

EP 0 309 898 A2

**Method for the induction of a cytotoxic T cell response**

Cell-mediated immunity plays a crucial role in preventing many infections caused by bacteria, viruses, fungi and parasites, in the mediation of the rejection of tumor cells and tissue grafts and in the pathogenesis of tissue injury in a number of diseases. Cell-mediated immunity is induced after the interaction of antigen with thymus derived lymphocytes (also called T-lymphocytes or T cells) leading to the generation of helper and cytotoxic T cells and subsequently to the production of a wide variety of lymphokines.

Antigens are recognized by T cells only in association with surface glycoproteins encoded by genes of the major histocompatibility complex (MHC) (Zinkernagel et al., Nature 248, 701-702 [1974]; Bevan, J. Exp. Med. 142, 1349-1364 [1975] and Katz et al., J. Exp. Med. 137, 1405-1418 [1973]). T helper cells (CD4[+] T lymphocytes), which co-operate with B cells in the generation of a humoral immune response against a soluble antigen, recognize antigens in association with the class II molecules of the MHC. It has clearly been shown that the antigen is first taken up into antigen presenting cells (APC, e.g. B-cells, macrophages, dentritic cells etc.) by endocytosis and is then degraded into peptides by peptidases, which are present in the lysosomes of these APC. The lysosomes containing the degraded antigen fuse with vesicles containing MHC class II molecules. The MHC class II molecules form a complex with the fragmented antigen, which complex is then transported to the surface of the APC. This complex can then be recognized by the T helper cells, which then are activated and "help" the B-cells to produce specific antibodies. (Schwartz, Ann. Rev. Immunol. 3, 237-261 [1985]). CD8[+] cytotoxic T lymphocytes (CTL), which are specific for cellular antigens, e.g. virally encoded proteins and transplantation antigens, recognize antigens in association with class I molecules of the MHC. They also recognize a fragmented form of the antigenic proteins. but in contrast to the situation described above for the helper cells, said antigenic proteins arise intracellularly, e.g., by transcription and translation of cellular genes or genes derived from a pathogenic agent. The antigenic protein is degraded in the cytoplasm. where peptide fragments are then picked up by MHC class I molecules. The complex of the MHC molecule with the peptide fragments is again transported to the surface of the cell. where it is presented to the cytotoxic T cells. The cell presenting the MHC/peptide fragement complex is then lyzed by the cytotoxic T cells specific for said complex of the MHC molecule and the peptide fragments (Germain, Nature 322, 687-689 [1986], Bevan, Nature 325, 192-194 [1987]). Thus T helper cells and cytotoxic T cells belong to two distinct lymphocyte populations that can be separated on the basis of their effector functions, as well as by the mechanism by which the antigenic polypeptide forms a complex with the MHC. The two forms of T cells make biological sense, e.g. during an attack of an aggressor like a virus, cytotoxic T cells would lyse the infected cells as foci of virus repliaction, whereas helper cells would guide the humoral res ponse to neutralize virus particles and toxins released by the infected cells.

It was believed that there was a clear separation between class I and class II restricted T lymphocyte populations, i.e. T lymphocytes reactive with derivates of cellular or soluble antigens, respectively, based on the two distinct mechanisms of intracellular transports of either class I or class II molecules. As discussed above vesicles containing class II MHC molecules can fuse with vesicles containing endocytosed digested soluble proteins. Class I molecules do not have access to this pathway and can only pick up peptides derived from the degradation of intracellular proteins. For example influenza nucleoprotein, which is known to be an antigen recognized in class I restricted fashion. is translated in the cytosol on free ribosomes and can, in its fragmented form, be recognized by CD8[+] cytotoxic T lymphocytes (Townsend et al., Cell 42. 457-467 [1985], Cell 44. 959-968 [1986] and Nature 324, 575-577 [1986]). Other evidence suggests that peptides derived from normal integral membrane proteins can also be presented in association with class I MHC molecules, as shown for MHC protein fragments (Maryanski et al., Nature 324, 578-579 [1986]; Parham et al., Nature 325. 625-628 [1987]).

The question whether there really is a clear distinction between the two forms of presentation of foreign antigens, namly the endocytic pathway for soluble antigen and the cytoplasmic pathway for internal antigen, was reexamined. Mice immunized with soluble ovalbumin as an antigen had previously been defined as a model for class II restricted antigen presentation and recognition (Shimonkevitz et al., J. Immunol. 133, 2067-2074 [1984]; Buus et al., Science 235. 1353-1358 [1987]). Using this model a novel immunization protocol was established that resulted in ovalbumin reactive class I restricted CTL which are capable of lyzing syngeneic target cells which were pulsed with soluble ovalbumin or which had been transfected with the ovalbumin gene.

In. other words it was shown that cytotoxic T cells specific for ovalbumin can be induced by in vivo immunization with a soluble protein. This proves that soluble proteins can be taken up by cells and be

2

presented to cytotoxic T cells and disproves the predictions in the state of the art, namely that the antigenic protein had to be produced intracellularly by transcription and translation of cellular genes or of genes derived from pathogenic agents. Furthermore, it was shown, by transfecting the ovalbumin gene into the mouse lymphoma cell line EL4 which is available from the American Tissue Culture Collection, 12301 Parklawn Drive, Rockville, Maryland, U.S.A. (ATCC No. TIB 39), that intracellular protein degradation provides ovalbumin fragments, which are capable to form a complex with MHC class I molecules, which complex is then presented and can be recognized by cytotoxic T cells, or generally spoken that immunization with soluble proteins can, in fact, initiate a class I restricted cytotoxic immune response against proteins derived from within the cells, such as foreign cellular proteins (e.g. transplantation antigens, tumor antigens etc.) or proteins encoded in the genome of microorganisms or parasites.

Therefore the present invention relates to a process for the induction of a cytotoxic T cell response characterized in that said response is induced by a soluble antigen derived from a pathogenic agent. In the present invention the term "soluble antigen derived from a pathogenic agent" is used to denote a polypeptide of a pathogenic agent or a fragment thereof, preferably a fragment comprising at least 10 amino acids which is brought in contact with the extracellular environment of $CD8^+$ cytotoxic T cells. A pathogenic agent is defined as a disease causing principle, e.g. a foreign cellular protein (e.g. a transplantation antigen, a tumor antigen or an intracellular protein derived from tissue injury) or a microorganism or parasite causing a disease in animals, preferably in mammals, most preferably in humans. Examples of such microorganisms or parasites are viruses, bacteria or protozoa, particularly intracellular parasites, which cause a disease by first infecting a cell, leading to a pathological transformation of said cell. Such microorganisms or parasites are known and are described e.g. by Davis et al. in "Microbiology", 3rd ed., Harper International Edition). Some of these microorganisms or parasites are listed in Table I and some of the diseases involved are given in parenthesis [].

Table I

Intracellular Parasites

1. Protozoa

Leishmania

e.g.
Leishmania donovani [Kala Azar]

Plasmodium [Malaria]

e.g.
Plasmodium vivax
Plasmodium ovale
Plasmodium malariae
Plasmodium falciparum

Isospora [Coccidiosis]

e.g.
Isospora belli
Isospora hominis

Toxoplasma [Toxoplasmosis]

e.g.
Toxoplasma gondii


Trypanosoma

e.g.
Trypanosoma brucei [Nagana disease]
Trypanosoma cruci [Chagas disease]
Trypanosoma gambiense [Sleeping disease]
Trypanosoma rhodesiense [Sleeping disease]


## 2. Fungi

e.g.
Cryptococcus neoformans [Cryptococcidosis]
Blastomyces dermatitidis [Blastomycosis]
Histoplasma capsulatum [Histoplasmosis]
Coccidioides immitis [Coccidioidomycosis]
Paracoccidioides brasiliensis
[Paracoccidioidomycosis]
Aspergillus fumigatus [Aspergillosis]
Sporothrix schenckii [Sporotrichosis]


## 3. Bacteria


Mycobacteria

e.g.
Mycobacterium tuberculosis [Tuberculosis]
Mycobacterium bovis [Tuberculosis]
Mycobacterium avium-intracellare [atypical Tuberculosis]
Mycobacterium kansasii [atypical Tuberculosis]
Mycobacterium leprae [Leprosy]
Mycobacterium ulcerans [Buroli Ulcer]


Salmonella

e.g.
Salmonella typhi [Typhoid fever]


Brucellae

e.g.
Brucellae abortus [Abort]
Brucellae melitensis

Corynebacteria

e.g.
Corynebacteria diphtheriae [Diphtheria]

Streptococci

e.g.
Streptococcus pneumoniae [Pneumonia]

4. Viruses

Picornaviridae

e.g.
Coxsackieviruses A and B
Echovirus
Rhinovirus [Common cold]
Polioviruses [Poliomyelitis]
Foot-and-mouth disease virus [Foot-and mouth disease]

Reoviridae

e.g.
Reovirus [Respiratory and Gastrointestinal Diseases]
Orbivirus [Colorado Tick Fever]
Rotavirus [Diarrhea, Fever]

Togaviridae

e.g.
Alphavirus [Encephalitis]
Flavivirus [Encephalitis]
Rubivirus [German Measles]

Orthomyxoviridae

e.g.
Influenzaviruses, types A, B, and C [Influenza]

Paramyxoviridae

e.g.
Parainfluenzavirus [Influenza, Pneumonia]
Mumpsvirus [Mumps]
Morbilivirus [Measles]
Respiratory syncytial virus [Bronchitis, Croup]

Rhabdoviridae

e.g.
Lyssavirus (e.g. rabies virus. Duvenhaga virus, Lagos bat virus, Mokola shrew virus)
Vesiculovirus (e.g. Chandipura virus)
Marburg virus [Marburg virus disease]


Arenaviridae

e.g.
Tacaribe group of viruses
Lymphocytic choriomeningitis virus
Lassa virus [Lassa fever]


Coronaviridae

e.g.
Coronavirus [Common cold, Pharyngitis]


Bunyaviridae

e.g.
California encephalitis virus [Encephalitis]


Parvoviridae

e.g.
Adenosatellavirus
Norwalk agent [Gastroenteritis]


Adenoviridae

e.g.
Adenovirus [Respiratory tract infections, Eye infections]
Adeno-associated viruses


Herpetoviridae

e.g.
Herpes simplex virus [Fever blisters]
Herpes zoster virus [Chickenpox, Shingles]
Cytomegalovirus
Epstein-Barr virus [Infectious Mononucleosis]


Pox viridae

e.g.
Variola virus [Smallpox]
Vaccinia virus
Molluscum contagiosum Virus [Molluscum contagiosum]

Cowpox virus [Cowpox]
Paravaccinia virus [Milker's nodules]
Orf virus [Contagious Pustular Dermatitis]

Hepatitis viridae

e.g.
Hepatitis virus A, B and C [Hepatitis]

RNA Tumor Viruses

e.g.
Leukosis virus
Sarcoma virus
Leukemia virus
Human immunodeficiency virus [AIDS]
Human T-cell leukemia virus [T-cell Leukemia]

DNA Tumor Virus

e.g.
Papovavirus (e.g. Polyoma Viruses SV40,
BK Virus JC Virus and Papilloma Virus [Warts])
Oncogenic Herpes Virus
Oncogenic Adenovirus
Oncogenic Poxvirus (e.g. Fibroma virus)

The polypeptides used as a soluble antigen in the present invention may be any polypeptide present in the microorganism or parasite, be it a surface polypeptide which is exposed to the outside of these pathogenic agents or be it a polypeptide which is not exposed to the outside of these pathogenic agents, that means a polypeptide which is not recognized as a surface antigen such as an internal polypeptide and fragments thereof. Examples for internal polypeptides are internal membrane proteins, cytoplasmic or nuclear proteins of protozoa, periplasmic, internal membrane proteins and cytoplasmic proteins of bacteria and internal envelope or core proteins of viruses. This is in contrast to polypeptides to be used as antigens for eliciting a humoral immune response against a pathogenic agent, because in this case the polypeptide can only be a surface polypeptide exposed to the outside of the pathogenic agent.

The polypeptides used as a soluble antigen in the present invention may have a disrupted tertiary structure or may be fragmented into smaller peptides. These peptides may or may not be exposed on the surface of the native polypeptide. This is again in contrast to polypeptides useful as antigens for eliciting a humoral immune response against a pathogenic agent, because in this case the polypeptide can only be a polypeptide fragment or antigenic determinant exposed to the outside of the pathogenic agent and it must have an intact tertiary structure. In certain cases a polypeptide may maintain its tertiary structure when it is fragmented into a small number of peptides. Only these fragments of the polypeptide corresponding to determinants which are exposed on the surface of the native polypeptide will induce a humoral immune response (Walter et al., in "Genetic Engineering"-Principles and Methods,Vol. 5, 61-91 [1983], J.W. Setlow, A. Hollaender eds., Plenum Press, New York).

It is known that a number of pathogenic agents constantly modify the structure of their surface polypeptides. This allows them to escape the attack by the immune system. The internal polypeptides on the other hand are much less variable. They are mostly structural proteins or enzymes, which would rapidly lose their biological activity or function when their structure would be modified constantly.

The advantages of the present invention can therefore be summarized as follows:

A cytotoxic T-cell response may be induced, by any polypeptide or polypeptide fragment of the pathogenic agent even when it has a disrupted tertiary structure, whereas an effective humoral immune response is induced only by surface polypeptides, viz. by polypeptides which are known often to change their structure, and only by determinants exposed to the surface of these polypeptides.

7

In order for a cytotoxic T cell response to be induced, the antigen has to be presented to the immune system in the context of class I proteins of the major histocompatibility complex (MHC).

The polypeptide used as a soluble antigen in the present invention may be isolated from the pathogenic agent and the fragments may be prepared chemically (e.g. by treatment with cyanogen bromide) or enzymatically (e.g. by peptidases) if desired. Alternatively the polypeptide or a fragment thereof may also be prepared synthetically based on structural information derived from the isolated pathogenic agent, e.g. the amino acid sequence of the polypeptide or the nucleotide sequence encoding it. The amino acid sequence of the polypeptide derived from the pathogenic agent may be determined by first isolating said polypeptide and then sequencing it by methods known in the art (see for example Hunkapiller et al., Methods in Enzymology 91, 399-413 [1983]).

Once the amino acid sequence of the polypeptide used as a soluble antigen in the present invention is known, the polypeptide can be chemically synthesized using known procedures for the formation of a peptide linkage between amino acids. Such procedures include, for example, any solution phase procedure permitting a condensation reaction between the free alpha amino group of an amino acid or residue thereof having its carboxylic group or other reactive groups protected and the free or activated primary carboxylic group of another amino acid or residue thereof having its amino group or other reactive groups protected.

Furthermore, such conventional procedures for synthesizing the polypeptide include any solid phase peptide synthesis method (Merrifield, J. Am. Chem. Soc. 85, 2149-2154 [1963]). In such a method the synthesis is commenced from the C-terminal end of the peptide by coupling a protected amino acid to a suitable resin. A starting material can be prepared by attaching an amino-protected amino acid via a benzyl ester linkage to a chloromethylated resin or a hydroxymethyl resin or via an amide bond to a benzhydrylamine (BHA) resin, a methylbenzhydrylamine (MBHA) resin or a benzyloxybenzyl alcohol resin. These resins are available commercially, and their preparation and use are well known.

General methods for protecting and removing protecting groups from amino acids which can be used in this invention are described in "The Peptides", Vol. 2 (E. Gross and J. Meienhofer, Eds.) Academic Press, New York, pp. 1-284 (1979). Protecting groups include, e.g., the 9-fluorenylmethoxycarbonyl (Fmoc), tert-butyloxycarbonyl (Boc), benzyl (Bzl), t-butyl (But), 2-chlorobenzyloxycarbonyl (2Cl-Z), dichlorobenzyl (Dcb) and 3,4-dimethylbenzyl (Dmb) groups.

After removal of the $\alpha$-amino protecting group from the initial (C-terminal) amino acid, the remaining protected amino acids are coupled step-wise in the desired order. The entire polypeptide may be synthesized in this way. Alternatively, small peptides may be constructed which are later joined, to give the final polypeptide. Appropriate coupling reagents are known in the art, with dicyclohexylcarbodiimide (DCC) being particularly suitable.

Each protected amino acid or peptide is introduced into the solid phase reactor in excess, and the coupling may be carried out in a medium of dimethylformamide (DMF) or methylene chloride ($CH_2Cl_2$), or mixtures thereof. In cases where incomplete coupling occurs, the coupling procedure is repeated before removal of the N$\alpha$-amino protecting group prior to the coupling of the next amino acid. The success of the coupling reaction at each stage of synthesis may be monitored. A preferred method of monitoring the synthesis is by the ninhydrin reaction. The coupling reactions and washing steps can be performed using automated instrumentation.

Cleavage of the peptide from the resin can be effected using procedures well known in peptide chemistry. For example, reaction with hydrogen fluoride in the presence of p-cresol and dimethylsulfide at 0°C for 1 hour may be followed by a second reaction with hydrogen fluoride in the presence of p-cresol for 2 hours at 0°C or with trifluoroacetic acid/methylene chloride/anisole. Cleavage of peptides from chloromethylated or p-benzyloxybenzyl alcohol resin supports produces finished peptides having carboxyl groups at the C-termini. Cleavage of peptides from benzhydrylamine or methylbenzhydrylamine resins produces peptides having C-terminal amide groups.

Alternatively, the peptides of the present invention may be prepared by using methods known in the art of recombinant DNA technology, e.g., as described by Maniatis et al. in "Molecular Cloning - A Laboratory Manual", Cold Spring Harbor Laboratory [1982]. The nucleotide sequence coding for the polypeptide may be determined by translating the known amino acid sequence of the polypeptide using the genetic code. Due to the degeneracy of this code, there is a substantial freedom in the choice of codons for any given amino acid. The codons selected can be adapted to the preferred codon usage of the host (Grosjean et al., Gene 18, 199-209 [1982]) used to express the recombinant polypeptide. Alternatively the nucleotide sequence may be determined by sequencing a nucleic acid (RNA or DNA) comprising the nucleotide sequence encoding said polypeptide using methods known in the art, e.g. the method of Maxam and Gilbert (Methods in Enzymology 65 (part 1), 497-559 [1980]) or of Sanger (Science 214, 1205-1210 [1981]).

A DNA fragment coding for the polypeptide used as a soluble antigen in the present invention may be chemically synthesized by conventional methods e.g. by the phosphotriester method which is described by Narang et al., Meth. Enzymol. 68, 90-108 [1979], or by the phosphodiester method as described by Brown et al., Meth. Enzymol. 68, 109-151 [1979]. In both methods oligonucleotides are prepared which can then be joined together to form the desired DNA fragment.

The DNA fragment coding for the polypeptide used as a soluble antigen in the present invention can also be isolated from the DNA of the pathogenic agent. This can be done by partially digesting said DNA with a suitable restriction endonuclease and selecting the desired fragment by methods known in the art (see Maniatis et al., supra). The DNA fragment may be operatively linked to an expression control sequence of an expression vector. A wide variety of such expression vectors are known.

The expression vector may then be inserted into a suitable host organism capable of expressing said polypeptide, using methods well-known in the art. The transformants are cultured under conditions permitting production of large amounts of polypeptide which may then be isolated and purified by methods known in the art.

The selection of a particular host is dependent upon a number of factors recognized by the art. These include, for example, compatibility with the chosen expression vector, toxicity of the encoded recombinant polypeptide to the host, ease of recovery of the desired polypeptide, expression characteristics, biosafety and costs. Within these general guidelines, examples of useful hosts are animal cells, yeast and gram-negative and gram-positive bacteria, especially strains of E. coli and B. subtilis. The transformed host obtained is then cultivated. The recombinant polypeptide produced by the host can then be isolated by methods known in the art.

The polypeptide can be purified by known methods, such as differential centrifugation, precipitation with ammonium sulfate, dialysis to remove salts (under normal or reduced pressure), gel filtration, preparative flat-bed iso-electric focusing, gel electrophoresis, various chromatographical methods, e.g., high performance liquid chromatography (HPLC), ion exchange chromatography, reverse phase chromatography and affinity chromatography, e.g., on dye bound carrier, on Sepharose coupled with monoclonal antibodies against said polypeptide, or on metal chelate resins (for a review see Sulkowski, Trends in Biotechn. 3, 1-7 [1985]) and the like.

The present invention further relates to vaccines for the induction of a cytotoxic T cell response and to the process for their preparation. The vaccines contain a soluble antigen derived from a pathogenic agent and a physiologically acceptable carrier.

Physiologically or pharmaceutically acceptable carriers are liquid media suitable for use as vehicles to introduce the soluble antigen into the patient. An example of such a carrier is saline solution. Peptides which are not soluble in saline solution may be solubilized by the addition of a pharmaceutically acceptable detergent or by adapting the pH of the solution, e.g. by adding hydrochloric acid, preferably to a final concentration of about 10 mM. The vaccine may be incorporated into a sterile container which is then sealed and stored at a low temperature, for example 4° C, or it may be freeze-dried.

The invention further relates to the use of a soluble antigen derived from a pathogenic agent for inducing a cytotoxic T cell response. In order to induce a cytotoxic T cell response one or more doses of the vaccine containing the soluble antigen suitably formulated may be administered. The injection of one dose has been found to be sufficient to induce a cytotoxic T cell response in a mouse. The doses used contained from less than 10 μg soluble antigen up to 200 mg of soluble antigen. This corresponds to a dose range of less than 35 mg up to 700 gr for a human assuming a body weight of 70 kg for the human and 20 gr for the mouse. Vaccinations are preferably done with the lowest amount of soluble antigen still capable of inducing a cytotoxic T cell response and therby preventing the pathogenic agent to cause the disease. In this way possible side-effects (e.g. toxicity) of the soluble antigen may be minimized.

Routes of administration, antigen dose, frequency of injections of the vaccine are all factors falling within the ordinary skill in the art. Preferably the vaccine used for the induction of a cytotoxic T cell response is given intravenously.

The vaccine may be administered with a pharmaceutically acceptable adjuvant. Typical adjuvants used for the vaccination of animals include but are not limited to Freund's complete or incomplete adjuvant (not suitable for human or livestock use), Adjuvant 65 (containing peanut oil, mannide monooleate and aluminum monostearate), and mineral gels such as aluminum hydroxide, aluminum phosphate and alum: surfactants such as hexadecylamine, octadecylamine, lysolecithin, dimethyldioctadecylammonium bromide. Further adjuvants are known to the skilled artisan (WHO Techn. Rep. Series 595, 1-40 [1976]; Jollis et al., "Chemical and Biological Basis of Adjuvants", in Molecular Biochemistry and Biophysics 13, 1-148 [1973] Springer Verlag Berlin).

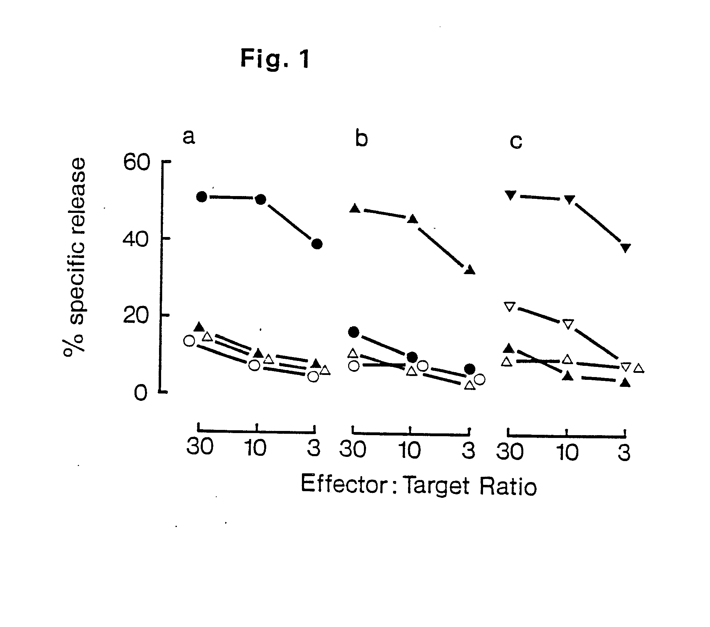

The present invention may be more readily understood by reference to the following examples when considered in connection with the accompanying Figures 1 to 3. These Figures are meant to show:

Figure 1

Specificity of secondary in vitro cultures after in vivo immunization with soluble ovalbumin. Cultures derived from C57BL6 (a and c) or DBA/2 (b) mice that had been immunized with ovalbumin in the presence (a and b) or absence (c) of syngeneic feeders were tested for their lytic activity on EL4 (C57BL6, ●o ▼ ▽) and P815 (DBA/2, ▲△) tumor targets in the presence (● ▼ ▲) or absence (o▽△) of ovalbumin fragmented by trypsin digestion (a and b) or treated with CNBr (c).

Figure 2

Specificity of a cloned cytotoxic T lymphocyte line specific for CNBr-fragmented ovalbumin. The killing activity of C11 was tested against EL4 (a), P815 (b) and S.AKR (AKR/J, c) in the presence (●) and absence (o) of CNBr-fragmented ovalbumin.

Figure 3

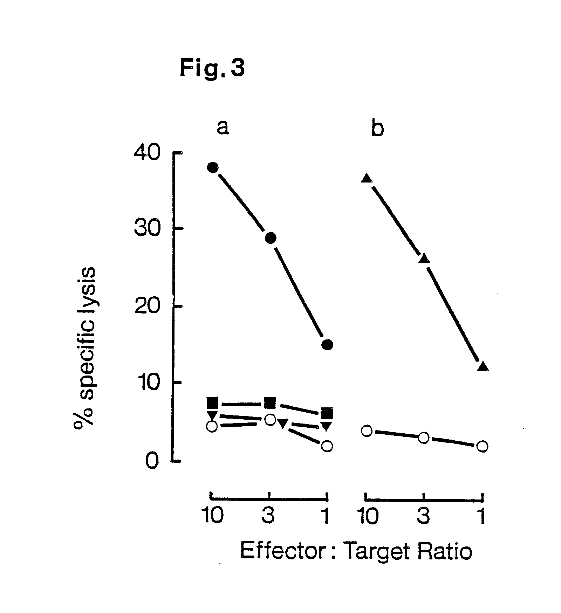

Ovalbumin-transfected target cells can be lysed by the cloned CTL C11. The susceptibility of lysis of the parental line EL4 (o, ●, ■, ▼ ) in the presence of CNBr-fragmented (●), trypsin-digested (■) whole ovalbumin ( ▼ ) or uncoated (o) shown in Fig. 3a was compared to the ovalbumin transfected line EL4, Ova4-17 (▲) shown in Fig. 3b.

It should be understood that the following examples are for illustrative purposes only and should not be construed as limiting this invention in any way to the specific embodiments recited therein.

## Example 1

$2 \times 10^6$ syngeneic irradiated (2000 rad) spleen cells were incubated in 0.5 ml phosphate buffered saline (PBS) containing 4 mg/ml ovalbumin (Sigma Fine Chemicals, St. Louis, USA) for 30 min on ice. 6 weeks old C57BL6 and DBA/2 (IFFA Credo, L'Arbresle, France) mice were then intravenously injected either with this suspension or with 0.5 ml of a solution of 0.2 mg/ml ovalbumin in PBS. After 7 days animals were sacrificed, spleens removed and cultured in single cell suspensions in flat-bottomed 24 well plates (Costar, Cambridge, USA) at $1 \times 10^7$ cells/ml in Iscove's modification of Dulbecco's medium (IMDM) (Gibco, Paisley, GB) supplemented with 10% fetal calf serum (Boehringer Mannheim, Rotkreuz, Switzerland), 2 mM glutamine, non-essential amino acids, 1 mM hydroxypyruvate, penicillin at 100 international units/ml, streptomycin at 100 μg/ml and gentamycin at 50 μg/ml (Sigma). Spleen cells obtained from animals immunized with feeders were grown for 5 days in the presence of soluble undegraded ovalbumin at 0.7 mg/ml, to the other cultures CNBr-fragmented ovalbumin (= cOVA) was added at 0.1 mg/ml. Ovalbumin was fragmented by CNBr (Sigma) according to Gross et al. (J. Biol. Chem. 237, 1856-1860 [1962]). The preparation was checked for complete degradation by gel filtration. For the cell mediated lympholysis (CML) assay, $^{51}$Cr-labeled target cells that do not express class II molecules (e.g. EL4 [ATCC No. TIB 39], P815 [ATCC No. TIB 64] or S.AKR) were preincubated with or without fragmented ovalbumin (either cOVA or ovalbumin digested by trypsin (Sigma) (= tOVA)) which was prepared as described by Shimonkevitz et al., J. Exp. Med. 158, 303-316 [1983]) at 0.3 mg/ml in supplemented IMDM for 60 min at 37° C. For the actual test $1 \times 10^4$ target cells suspended in the same medium were incubated for 4 1/2 hours in round-bottomed microtiter plates (total volume = 0.2 ml) (Costar) at effector to target ratios ranging from 30:1 to 1:1. Percent specific lysis of these target cells was calculated as described by Townsend et al. (Cell 42, 457-467 [1985]).

As demonstrated in Figure 1, neither of the two immunization protocols resulted in appreciably lytic activity towards allogeneic targets in the presence or absence of ovalbumin preparations. However, C57BL6 (H-2$^b$) and DBA/2 (H-2$^d$) mice immunized with ovalbumin mounted a specific response to syngeneic targets, EL4 (H-2$^b$) or P815 (H-2$^d$), respectively, in the presence of their specific antigen, whereas in the absence of specific antigen only minor background activity could be observed. The presence of syngeneic feeder cells

10

at the time of immunization had no effect on the activity of the responding population. Fragments of other proteins, for example pigeon cytochrome c or hen egg lysozyme (HEL) could not substitute for cOva or tOva. Similar results were obtained with blast cells as targets excluding any peculiar properties of the tumor cells.

## Example 2

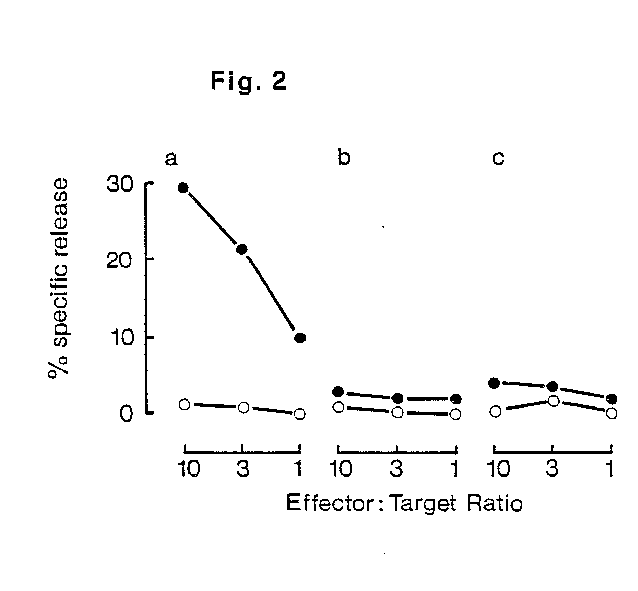

Example 1 shows that a soluble protein can elicit a specific lytic response suggestive for MHC class I restricted recognition. The cell population most likely responsible for this reactivity was further characterized. From a secondary in vitro culture directed against cOVA, a cloned cytotoxic T lymphocyte line, C11 (C57BL/6 derived), was established by conventional limiting dilution. C11 has been carried by weekly restimulation with 0.1 mg/ml cOVA and syngeneic irradiated spleen cells in supplemented IMDM plus an exogenous source of IL-2. As determined by indirect surface immunofluorescence, C11 has the phenotype of a conventional CTL: CD1$^+$, CD4$^-$, CD8$^+$. The CML assay was performed as described in Example 1. As depicted in Figure 2, C11 demonstrated the same restriction pattern as the uncloned population. The syngeneic target EL4 was susceptible to lysis only in the presence of cOVA, otherwise it was not killed by C11, nor were allogeneic targets. These findings indicate that the immunization protocols induce a MHC class I restricted response that is, at least in part, carried by conventional CD8$^+$ killer cells.

## Example 3

In order to examine whether the immune response against ovalbumin measured in Examples 1 and 2 is directed only against epitopes on the ovalbumin molecule that have been obtained by chemical or enzymatic fragmentation, or whether the immune response is also directed against epitopes obtained by intracellular protein degradation within the target cell, EL4 cells (ATCC No. TIB 39) were transfected with the plasmid BOVAneo capable of expressing ovalbumin cDNA. BOVAneo was obtained by introducing the ovalbumin cDNA (McReynolds et al., Nature 273, 723-728 [1978]) into the BM6neo expression vector. The BM6neo expression vector provides the dominant selectable marker for resistance to the antibiotic G418. Alternatively the vector pSV2-neo (ATCC No. 37149) described by Southern et al. (J. Molec. Appl. Genet. 1, 327-341 [1982]) or another expression vector containing said selectable marker may be used. G 418 resistant clones were selected and ovalbumin expression was determined by intracellular indirect immunofluorescence using a monoclonal mouse anti-ovalbumin antibody. The cell line EL4.Ova4-17 was picked, subcloned by limiting dilution and tested for susceptibility for lysis mediated by C11. The extent of lysis of the parental cell line in the presence of cOVA (Fig. 3a) is comparable to the transfected cell line (Fig. 3b). In contrast, C11 could not kill EL4 grown for 18 hrs in medium containing 10 mg/ml soluble undigested oval bumin, excluding the possibility that secreted ovalbumin was taken up by EL4 and then presented in conjunction with MHC. It is noteworthy, however, that trypsin digestion of ovalbumin seems to destroy the epitope recognized by C11.

The results presented show that MHC class I responses can be raised against a soluble protein like ovalbumin. That means that a mechanism exists, that allows exogenous antigens to be presented in conjunction with MHC class I molecules similar to antigens expressed within the cell. The fact that EL4 grown in soluble ovalbumin cannot be lysed by C11, in contrast to its counterpart transfected with the ovalbumin gene points to the existence of a specialized antigen presenting cell responsible for the induction of CTL. However, in contrast to Bevan et al. [1987], supra, this antigen presenting cell can take up soluble material.

## Example 4

The experiment described in Example 1 was repeated using hen egg lysozyme (HEL) as antigen. HEL reactive class I restricted CTL effector cells were generated in C57BL/6-mice. The animals were sacrificed, and spleen cells were cultured for 7 days in the presence of 100 μg/ml HEL at a effector/target-ratio of 20:1. The results are summarized in Table II.

Table II

| Target | HEL | Lysis |
|--------|-----|-------|
| EL4 | - | -2% |
| EL4 | + | 10.5% |
| P815 | + | -1% |
| S.AKR | + | 0% |

## Claims

1. A method for the induction of a cytotoxic T cell response characterized in that said response is induced by a soluble antigen derived from a pathogenic agent.

2. A vaccine to be used for the induction of a cytotoxic T cell response characterized in that it contains a soluble antigen derived from a pathogenic agent.

3. A method for the preparation of a vaccine used for the induction of a cytotoxic T cell response characterized in that a soluble antigen derived from a pathogenic agent is combined with a physiologically acceptable carrier.

4. Use of a soluble antigen derived from a pathogenic agent for inducing a cytotoxic T cell response.

5. The invention as hereinbefore described.

# Fig. 1

# Fig. 2

Fig. 3